(19) Europäisches Patentamt
European Patent Office
Office européen des brevets

(11) **EP 4 656 712 A1**

(12) **EUROPEAN PATENT APPLICATION**
published in accordance with Art. 153(4) EPC

(43) Date of publication:
**03.12.2025 Bulletin 2025/49**

(21) Application number: **24747219.4**

(22) Date of filing: **19.01.2024**

(51) International Patent Classification (IPC):
*C12M 3/00* (2006.01)   *C12N 5/071* (2010.01)
*C12N 5/09* (2010.01)   *C12Q 1/06* (2006.01)

(52) Cooperative Patent Classification (CPC):
**C12M 3/00; C12N 5/06; C12Q 1/06**

(86) International application number:
**PCT/JP2024/001432**

(87) International publication number:
**WO 2024/157891 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.01.2023 JP 2023008762**

(71) Applicant: **Toppan Holdings Inc.**
**Tokyo 110-0016 (JP)**

(72) Inventors:
• **TSUKAMOTO, Kei**
  **Tokyo 110-0016 (JP)**
• **SUZUKI, Mizuho**
  **Tokyo 110-0016 (JP)**
• **KITANO, Shiro**
  **Tokyo 110-0016 (JP)**

(74) Representative: **Plasseraud IP**
**104 Rue de Richelieu**
**CS92104**
**75080 Paris Cedex 02 (FR)**

(54) **CELL-CONTAINING CONTAINER**

(57) The cell-containing vessel includes: a cell culture vessel; a cell culture medium contained in the cell culture vessel; a first cell layer located on a cell culture surface of the cell culture vessel; and a second cell layer that contains stroma-forming celles and is located on the first cell layer. The second cell layer is removable from the first cell layer.

FIG.1

**Description**

[Technical Field]

The present invention relates to a cell-containing vessel. More specifically, the present invention relates to a cell-containing vessel, a method for culturing cells, and a method for evaluating an effect of a drug on a cell.
The present application claims the benefit of priority from Japanese Patent Application No. 2023-008762 filed on January 24, 2023, the contents of which are incorporated herein by reference.

[Background Art]

Conventionally, cancer research has focused on experiments using established cell lines that have been subcultured under optimized conditions. However, the properties of cancer cell lines that have been maintained and cultured outside of the body for many years change from those of the original patient tumor tissue, and they may exhibit behaviors that do not adequately reflect those observed in the body. To address this issue, primary culture of cancer cells is seen as promising for higher-precision anti-cancer drug development and selection of the optimal treatment for each patient.
For example, NPL 1 describes CD-DST (Collagen gel droplet embedded drug sensitivity test) using cells from primary culture. This test is a drug susceptibility test in which tissue or cells isolated from a patient are embedded in collagen gel and cultured for verification. However, it cannot be said that a culture method for primary culture cells is established, and they are prone to culture failure.
Methods for primary culture of cancer cells from patient tumor tissue have been proposed, such as a method in which Y-27632, a ROCK inhibitor, is added to a culture medium to inhibit cell death (also known as apoptosis) associated with cell dispersion (NPL 2) and a method in which cell aggregates of a certain size are obtained while maintaining intercellular adhesion and cultured in suspension (PTL 1). These culture methods use a serum-free medium for stem cells supplemented with a serum substitute and various growth factors. However, serum-free media for stem cells are generally expensive. In addition, in a growth environment where a large amount of growth factors is artificially added, signaling pathways different from those in the actual body may be upregulated or downregulated. In such an environment, results different from those obtained in the actual body may be obtained, especially in susceptibility tests using molecularly targeted drugs.
PTL 2 describes a method for primary culture of cells in tissue (also referred to as biological tissue) collected from a living body. The method uses a culture medium generally used for cell culture without adding growth factors or inhibitors.

[Citation List]

[Patent Literature]

[PTL 1] JP 5652809 B
[PTL 2] WO 2019/039457 A

[Non Patent Literature]

[NPL 1] Takamura Y. et al., Prediction of chemotherapeutic response by collagen gel droplet embedded culture-drug sensitivity test in human breast cancers, Int. J. Cancer, vol. 98, 450-455, 2002.
[NPL 2] Zhang L. et al., ROCK Inhibitor Y-27632 Suppresses Dissociation-Induced Apoptosis of Murine Prostate Stem/Progenitor Cells and Increases Their Cloning Efficiency, PLoS ONE, vol. 6, e18271, 2011.
[NPL 3] Nishiguchi A., et al., Cell-cell crosslinking by bio-molecular recognition of heparin-based layer-by-layer nanofilms, Macromol Biosci., vol. 15, 312-317, 2015.

[Summary of the Invention]

[Technical Problem]

In the method described in PTL 2, cells from tissue taken from a living body (also referred to as primary cells) are cultured by being seeded on the top surface of a cell structure containing cells forming the stroma (also referred to as stromal cells). To culture primary cells for a long period of time using this method, it is necessary to exchange the cell structure containing stromal cells when subculturing the cells. However, the inventors found that the method described in PTL 2 requires a lot of work to separate the primary cells from the cell structure containing stromal cells. Therefore, the method described in PTL 2 leaves room for improvement in long-term culture of primary cells.

An object of the present invention is to provide a technique for long-term culture of primary cells using a general cell culture medium without adding growth factors or inhibitors. The present invention can also be applied to cells other than primary cells.

[Solution to Problem]

The present invention includes the following aspects.

[1] A cell-containing vessel including: a cell culture vessel; a cell culture medium contained in the cell culture vessel; a first cell layer located on a cell culture surface of the cell culture vessel; and a second cell layer that contains stroma-forming celles and is located on the first cell layer, the second cell layer being removable from the first cell layer.
[2] The cell-containing vessel according to [1], wherein the first cell layer contains primary cells.
[3] The cell-containing vessel according to [1] or [2], further including a cylindrical member having openings at both ends, wherein the cylindrical member is contained in the cell culture vessel so that one of the openings is in contact with the first cell layer, and the second cell layer is placed inside the cylindrical member.
[4] The cell-containing vessel according to any one of [1] to [3], wherein the second cell layer has been gelled.
[5] A method for culturing cells, including: incubating the cell-containing vessel according to any one of [1] to [4], and exchanging the second cell layer to subculture cells contained in the first cell layer.
[6] A method for evaluating an effect of a drug on a cell, the method comprising:
incubating the cell-containing vessel according to any one of [1] to [4] in the presence of the drug, exchanging the second cell layer to subculture cells contained in the first cell layer, and evaluating the effect of the drug on the cells contained in the first cell layer.

[Advantageous Effects of the Invention]

According to the present invention, it is possible to provide a technique for long-term culture of primary cells using a general cell culture medium without adding growth factors or inhibitors. The present invention can also be applied to cells other than primary cells.

[Brief Description of the Drawings]

Fig. 1 is a schematic cross-sectional view illustrating the structure of an example of a cell-containing vessel.
Fig. 2 is a schematic cross-sectional view illustrating an example of a method of producing the second cell layer (cell structure) containing stroma-forming celles.

[Description of the Embodiments]

[Cell-containing vessel]

In one embodiment, the present invention provides a cell-containing vessel including: a cell culture vessel; a cell culture medium contained in the cell culture vessel; a first cell layer located on a cell culture surface of the cell culture vessel; and a second cell layer that contains stroma-forming cells and is located on the first cell layer, the second cell layer being removable from the first cell layer.

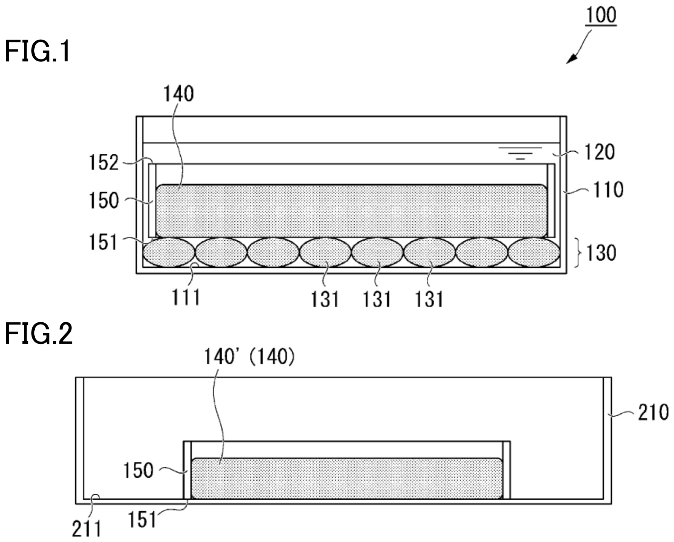

Fig. 1 is a schematic cross-sectional view illustrating the structure of an example of a cell-containing vessel according to the present embodiment. As shown in Fig. 1, a cell-containing vessel 100 includes a cell culture vessel 110, a cell culture medium 120 contained in the cell culture vessel 110, a first cell layer 130 that contains cells 131 and is located on a cell culture surface 111 of the cell culture vessel 110, and a second cell layer 140 that contains cells forming the stroma and is located on the first cell layer 130. The second cell layer 140 is removable from the first cell layer 130.
As in the example of Fig. 1, the cell-containing vessel 100 further has a cylindrical member 150 having openings 151, 152 at both ends. The cylindrical member 150 is contained in the cell culture vessel 110 so that one opening 151 is in contact with the first cell layer 130. The second cell layer 140 may be placed inside the cylindrical member 150. The details of the cylindrical member 150 will be described later.

As will be described later using Examples, according to the cell-containing vessel of this embodiment, it is possible to achieve a long-term culture of primary cells using a general cell culture medium without adding growth factors or inhibitors. That is, the cells 131 contained in the first cell layer 130 may be primary cells or cells other than primary cells. An example of a cell other than primary cells is an established cell line.

Being possible to achieve long-term culture of primary cells means that the primary cells can be grown for, for example, 5 days or more, preferably 10 days or more, more preferably 20 days or more, and even more preferably 30 days or more. Although the upper limit of the period for long-term culture of primary cells is not particularly limited, it may be, for example, 180 days.

(Cell culture vessel)

The cell culture vessel 110 is not particularly limited, and any vessel commonly used for cell culture can be used. Specific examples of the cell culture vessel 110 include dishes and well plates. The well plate may be, for example, a 6-well, 12-well, 24-well, 48-well, or 96-well plate. The cell culture surface 111 of the cell culture vessel 110 may be a flat bottom, or may have a lattice- or honeycomb-shaped uneven structure formed thereon.

(Cylindrical member)

The cylindrical member 150 is not particularly limited as long as it allows construction of the second cell layer 140 (hereinafter also referred to as a "cell structure") containing cells from the stroma and allows culturing the constructed cell structure. For example, the cylindrical member 150 may be a cell culture insert (for example, a Transwell (registered trademark) insert, a Netwell (registered trademark) insert, a Falcon (registered trademark) cell culture insert, or a Millicell (registered trademark) cell culture insert), a tube, or a pipe.

The cylindrical member 150 has openings at both ends. At least a part of the contents of the cylindrical member 150 (for example, components secreted from cells) can be put into and extracted from the cylindrical member 150 through the openings. For example, semipermeable membrane (also referred to as a membrane) may be provided in one or both openings of the cylindrical member 150. Alternatively, a cell culture insert from which the membrane usually placed at the bottom has been removed may be used as the cylindrical member 150.

A handle may be provided on the upper part of the cylindrical member 150 for attaching/detaching it to/from a dish or a well plate. It is also possible to provide a magnet on the upper part of the cylindrical member 150 so that the cylindrical member 150 can be extracted by magnetic force.

The area of the cross section of the cylindrical member 150 perpendicular to its axial direction should be smaller than the bottom area of the cell culture vessel 110 (also referred to as the area of the cell culture surface 111) so that it can be accommodated inside the cell culture vessel 110. Here, the cylindrical member 150 is placed in the cell culture vessel 110 so that one opening 151 comes into contact with the cell culture surface 111 of the cell culture vessel 110. Alternatively, the cylindrical member 150 is placed in the cell culture vessel 110 so that one opening 151 comes into contact with the first cell layer 130 located on the cell culture surface 111.

(Cell culture medium)

The cell culture medium 120 in the cell culture vessel of this embodiment can be a general cell culture medium that does not contain growth factors or inhibitors of signal transduction pathways. The medium is not particularly limited, and examples thereof include media obtained by adding approximately 1 to 20 vol% serum to a basal medium such as DMEM, EMEM, MEM$\alpha$, RPMI-1640, McCoy's 5A, or Ham's F-12. Examples of the serum include calf serum (CS), fetal bovine serum (FBS), and fetal horse serum (HBS).

(Primary cells)

Primary cells refer to cells taken directly from biological tissue. Primary cells are considered to closely reflect the properties of the original biological tissue. Culturing primary cells is called primary culture. In primary culture, a plurality of types of cells contained in the biological tissue may be cultured simultaneously, or only a specific type of cell may be isolated from the cells contained in the biological tissue and cultured.

The biological tissue from which the primary cells are derived may be tissue taken from any animal species. For example, the biological tissue may be sampled from animals such as humans, monkeys, dogs, cats, rabbits, pigs, cows, mice, or rats.

The biological tissue may be solid tissue or liquid tissue. Examples of solid tissues include epithelial tissue, connective tissue, muscle tissue, nerve tissue, stromal tissue, and mucosal tissue obtained by surgical excision.

Examples of liquid tissues include bodily fluids such as blood, lymph, pleural fluid, ascites, cerebrospinal fluid, tears, saliva, and urine.

These tissues can be, for example, extracted with a scalpel or laser or sampled with an injector or swab during surgery or endoscopic examination. In the case of living human tissue, for example, tissue sampled for clinical testing can be used.

The primary cells may be cells derived from normal tissue or dysfunctional cells from diseased tissue or the like. For example, effective primary culture of cancer cells contained in tumor tissue collected from a cancer patient can be achieved. Note that cancer cells refer to cells that have been derived from somatic cells and that have acquired infinite proliferation potential. In primary culture, cancer cells may be primarily cultured together with cells other than cancer cells contained in tumor tissue collected from the cancer patient, or only cancer cells may be isolated and primarily cultured.

Cancer cells may be isolated with a common method, such as separation using a cell sorter, magnetic separation, dielectrophoresis, size fractionation, or density gradient fractionation. These methods may be used singly or in combination of two or more. The method for isolating cells can be appropriately determined based on the organ from which the original patient tumor was derived, the clinical background, or the results of various preceding tests.

When a cell sorter is used, cancer cells can be selected by staining them with a fluorescently labeled antibody or a fluorescent probe and isolating the stained cells. In addition, since cell sorters can determine whether cells are alive or dead based on the forward and side scattered light values, it is possible to more efficiently select and collect living cancer cells. When magnetic separation is used, cells are magnetically labeled using an antibody. Any of various methods can be selected, such as the positive selection method in which labeled cancer cells are magnetically collected, and the negative selection method in which cells other than the labeled cancer cells are magnetically removed.

Primary culture using the cell-containing vessel of this embodiment enables primary culture of disease-related cells collected from patients suffering from various diseases, such as cancer cells collected from cancer patients, with a high success rate. The cells obtained by the primary culture of disease-associated cells are particularly suitable for cell-based assays. The cell-containing vessel of this embodiment is also useful for establishing cultured cell lines of disease-related cells collected from patients. For example, by primary culture of the cells contained in a patient's tumor tissue using the cell-containing vessel of this embodiment, it is possible to efficiently establish a patient-derived cancer cell line that reflects the characteristics of the patient's tumor, such as proliferative capacity, better than a normal cell line.

Non-limiting examples of the cancer from which cancer cells to be subjected to primary culture are derived include breast cancer (e.g., invasive ductal breast cancer, ductal carcinoma in situ, and inflammatory breast cancer), prostate cancer (e.g., hormone-dependent prostate cancer and hormone-independent prostate cancer), pancreatic cancer (e.g., pancreatic duct cancer), gastric cancer (e.g., papillary adenocarcinoma, mucinous adenocarcinoma, and adenosquamous carcinoma), lung cancer (e.g., non-small-cell lung cancer, small-cell lung cancer, and malignant mesothelioma), colon cancer (e.g., gastrointestinal stromal tumor), rectal cancer (e.g., gastrointestinal stromal tumor), colorectal cancer (e.g., familial colorectal cancer, hereditary non-polyposis colorectal cancer, and gastrointestinal stromal tumor), small intestinal cancer (e.g., non-Hodgkin's lymphoma and gastrointestinal stromal tumor), esophageal cancer, duodenal cancer, tongue cancer, pharyngeal cancer (e.g., nasopharyngeal cancer, oropharyngeal cancer, and hypopharyngeal cancer), head and neck cancer, salivary gland cancer, brain tumor (e.g., pineal astrocytoma, pilocytic astrocytoma, diffuse astrocytoma, and anaplastic astrocytoma), neurilemmoma, liver cancer (e.g., primary liver cancer and extrahepatic bile duct cancer), renal cancer (e.g., renal cell cancer and transitional cell cancer of the renal pelvis and ureter), gallbladder cancer, bile duct cancer, pancreatic cancer, hepatoma, endometrial cancer, cervical cancer, ovarian cancer (e.g., epithelial ovarian cancer, extragonadal germ cell tumor, ovarian germ cell tumor, and ovarian low-malignant potential tumor), bladder cancer, urethral cancer, skin cancer (e.g., intraocular (ocular) melanoma and Merkel cell carcinoma), hemangioma, malignant lymphoma (e.g., reticulosarcoma, lymphosarcoma, and Hodgkin's disease), melanoma (malignant melanoma), thyroid cancer (e.g., medullary thyroid cancer), parathyroid cancer, nasal cancer, paranasal cancer, bone tumor (e.g., osteosarcoma, Ewing's tumor, uterine sarcoma, and soft-tissue sarcoma), metastatic medulloblastoma, hemangiofibroma, dermatofibrosarcoma protuberans, retinal sarcoma, penile cancer, testicular tumor, pediatric solid cancer (e.g., Wilms tumor and pediatric renal tumor), Kaposi sarcoma, AIDS-associated Kaposi sarcoma, tumor of the maxillary sinus, fibrous histiocytoma, leiomyosarcoma, rhabdomyosarcoma, chronic myeloproliferative disorders, leukemia (e.g., acute myelogenous leukemia and acute lymphoblastic leukemia), and the like.

When the biological tissue is solid tissue, it is preferable to fragment it in advance so that the primary cells therein can efficiently adhere to the second cell layer 140 (also referred to as cell structure) containing the cells that form the stroma and be cultured. The fragmenting of the biological tissue is preferably mechanically carried out using scissors, a knife, a scalpel, tweezers, or the like, but is not particularly limited thereto. The biological tissue is preferably fragmented to, for example, approximately 5 mm or less to extract the primary cells inside the tissue more efficiently.

The biological tissue fragments can be directly subjected to primary culture, but it is also preferable to subject them to an enzymatic treatment. By performing the enzymatic treatment, the primary cells inside the fragments are more likely to be exposed on their surfaces so that they are more likely to come into contact with the cell structure. The enzyme treatment can also be carried out when the biological tissue is liquid tissue.

The enzyme used in the enzymatic treatment of the biological tissue or its fragments is not particularly limited; however, enzymes that degrade proteins, sugars, lipids, nucleic acids, and the like are preferably used. One or more enzymes may be used in the enzymatic treatment of biological tissue fragments. In the present invention, preferably one or more enzymes selected from the group consisting of trypsin, collagenase, dispase, elastase, papain, and hyaluronidase are used, more preferably collagenase or two or more enzymes including collagenase are used, and even more preferably collagenase and dispase are used together with one or more other enzymes as necessary. Note that the enzyme is not particularly limited as long as it has the desired enzymatic activity. It may be an enzyme derived from any biological species or an artificial enzyme modified from a natural enzyme. It may also be an enzyme extracted and purified from a cell, or a chemically synthesized enzyme.

In the fragmentation or enzymatic treatment of biological tissue, DNase I may be additionally used to prevent cells from aggregating into clumps due to the effect of DNA released from cells lysed during the fragmentation or enzymatic treatment. The DNase I used is not particularly limited as long as it is an enzyme having DNase I activity. Commercially available enzyme mixtures containing DNase I and other enzymes that degrade biological components such as proteins include Liberase Blendzyme 1 (registered trademark) (manufactured by Roche Diagnostics) and Tumor Dissociation Kit (manufactured by Miltenyi Biotec).

The enzyme treatment may be carried out at any temperature at which the enzyme can exert its enzymatic activity. The treatment temperature for the enzyme treatment is preferably 30 to 40°C, more preferably 37°C, in order to minimize the effect on cells in the biological tissue fragments. The treatment time for the enzyme treatment is not particularly limited but can be, for example, 10 to 90 minutes, preferably 30 to 60 minutes.

It is preferable to count the number of cells in the enzyme-treated biological tissue before seeding them on the cell culture surface 111 of the cell culture vessel 110. In particular, it is preferable to count the number of living cells. The counting of cells and counting of living cells can be carried out using a commonly used method. For example, the number of living cells can be counted by staining using trypan blue.

The biological tissue fragments may be washed with a buffer or the culture medium before the enzyme treatment. The buffer used for washing may be a phosphate buffer, an acetate buffer, a citrate buffer, a borate buffer, a tartrate buffer, a Tris buffer, or PBS. An antibiotic can also be added to the buffer or culture medium for washing. Particularly preferably, the tissue can be washed with a phosphate buffered saline (PBS) containing penicillin G (200 U/mL), streptomycin sulfate (200 $\mu$g/mL), and amphotericin B (0.5 $\mu$g/mL). The number of times washing is carried out can be appropriately determined depending on the origin of the collected biological tissue, but preferably it is 3 to 8 times. The tissue may be washed with a buffer or culture medium only after the enzyme treatment, or before and after the enzyme treatment.

When only specific primary cells in the biological tissue are to be cultured, only the primary cells of the desired cell type are extracted from the biological tissue or enzyme-treated biological tissue to be seeded on the cell culture surface 111 of the cell culture vessel 110. For example, after cancer cells are extracted from an enzyme-treated tumor tissue, only the cancer cells are seeded on the cell culture surface 111 of the cell culture vessel 110.

In a case where only cancer cells are extracted and cutured from biological tissue or enzyme-treated biological tissue derived from a cancer patient, the amount of cancer cells contained in the biological tissue or enzyme-treated biological tissue may be confirmed before extracting the cancer cells. Cancer cells can be extracted by using the expression of cancer cell-specific proteins or increased enzyme activity as a cancer marker. The cancer marker is not particularly limited. For example, when a protein that is specifically expressed in cancer cells, such as EpCAM, CEA, Cytokeratin, or HER2, is used as the cancer marker, the cancer cells can be visualized by immunohistochemistry (IHC) staining or immunofluorescence (IF) staining using an antibody against it. When the increased enzyme activity of $\gamma$-glutamyl transpeptidase or $\beta$-galactosidase in cancer cells is used as the cancer marker, its enzyme activity can be measured using a fluorescent probe such as ProteoGREEN (registered trademark, manufactured by Goryo Chemical, Inc.) or GlycoGREEN (registered trademark, manufactured by Goryo Chemical, Inc.).

As shown in Fig. 1, the cells 131 are seeded on the cell culture surface 111 of the cell culture vessel 110 and incubated to obtain the first cell layer 130 containing the cells 131.

(Stroma-forming celles)

The cells forming the stroma (also called stromal cells) which form the second cell layer 140 (also called cell structure) is not particularly limited. The cells may be cells obtained from an animal, cells obtained by culturing cells obtained from an animal, cells obtained by subjecting cells obtained from an animal to various treatments, or cultured cell lines. It is also possible to use commercially available cells or patient-derived cells. In the case of cells obtained from animals, the sampling site is not specifically limited. The cells may be somatic cells derived from bone, muscle, viscus, nerve, brain, bone, skin, blood, or the like; reproductive cells; or embryonic stem cells (ES cells). Moreover, the biological species from which the cells constituting the cell structure according to the present invention are derived is not limited. For example, usable cells can be derived from animals such as humans, monkeys, dogs, cats, rabbits, pigs, cows, mice, and rats. The cells obtained by culturing cells obtained from animals may be cells from primary culture or subcultured cells.

Further, the cells obtained by applying various treatments may include induced pluripotent stem cells (iPS cells) or cells after differentiation induction. The cell structure may be composed of only cells derived from the same biological species, or cells derived from several types of biological species.

Examples of the stromal cells include endothelial cells, fibroblasts, pericytes, immune cells, neural cells, mast cells, epithelial cells, cardiac muscle cells, hepatic cells, pancreatic islet cells, tissue stem cells, and smooth muscle cells. Immune cells are cells involved in immunity. Specific examples of the immune cells include lymphocytes, macrophages, and dendritic cells. Lymphocytes include T cells, B cells, NK cells, plasma cells, and the like. One or more types of stromal cells may be contained in the cell structure according to the present invention. The stromal cells contained in the cell structure according to the present invention preferably include one or more types selected from the group consisting of fibroblasts, pericytes, endothelial cells, and immune cells.

The number of stromal cells in the cell structure is not particularly limited; however, in order to produce a cell structure that more closely resembles stromal tissue, the abundance ratio of stromal cells to all the cells forming the cell structure (i.e., the abundance ratio of the number of endothelial cells to the total number of cells forming the cell structure) is preferably 30% or more, more preferably 50% or more, even more preferably 70% or more, and particularly preferably 80% or more. The upper limit of the abundance ratio of stromal cells to all the cells forming the cell structure may be, for example, 100%.

Vascular and lymphatic network structures are considered to be important for a cell structure to exhibit functions similar to those of in vivo stromal tissue. Therefore, the cell structure preferably includes a vascular network structure. Namely, the cell structure is preferably one in which a vascular network structure such as of lymphatic vessels and/or blood vessels is three-dimensionally produced inside a laminate of non-vascularized cells to produce tissues closer in structure to those in vivo. The vascular network structure may be formed only on the inside of the cell structure, or may be formed so that at least part thereof is exposed on the front surface or the bottom surface of the cell structure. Further, the vascular network structure may span the entire cell structure, or may be formed only in a specific cell layer. In the present specification, the term "vascular network structure" refers to a net-like structure, such as a vascular network or lymphatic network, in body tissue.

A vascular network structure can be formed by incorporating endothelial cells, which constitute vessels, as stromal cells. The endothelial cells contained in the cell structure may be vascular endothelial cells or lymphatic endothelial cells. Moreover, both vascular endothelial cells and lymphatic endothelial cells may be contained.

When the cell structure has a vascular network structure, the cells other than the endothelial cells in the cell structure are preferably cells that constitute surrounding tissue of vessels in a living body, because the endothelial cells can easily form a vessel network maintaining the original function and shape. A cell structure at least containing fibroblasts as cells other than endothelial cells is more preferable, to more closely resemble in vivo stromal tissue and its surrounding environment. It is even more preferable if the cell structure contains vascular endothelial cells and fibroblasts, lymphatic endothelial cells and fibroblasts, or vascular endothelial cells, lymphatic endothelial cells, and fibroblasts. Further, the cells other than endothelial cells contained in the cell structure may be derived from the same species as that of the endothelial cells, or from different species.

The number of endothelial cells in the cell structure is not specifically limited as long as it is sufficient for forming a vascular network structure, and can be determined as appropriate in consideration of the size of the cell structure, the types of endothelial cells and cells other than endothelial cells, and the like. For example, a cell structure having a vascular network structure can be prepared by setting the abundance ratio of endothelial cells to all the cells constituting the cell structure (i.e., the ratio of the number of endothelial cells to the total number of cells constituting the cell structure) to 0.1% or more. When fibroblasts are used as the cells other than endothelial cells, the number of endothelial cells in the cell structure is preferably 0.1% or more, and more preferably 0.1% to 5.0% of the number of fibroblasts. When both vascular endothelial cells and lymphatic endothelial cells are contained as endothelial cells, the total number of vascular endothelial cells and lymphatic endothelial cells is preferably 0.1% or more, and more preferably 0.1% to 5.0% of the number of fibroblasts.

The size and shape of the cell structure are not limited. In order to form a cell structure in a state closer to the in vivo stromal tissue and to achieve primary culture in an environment that is more likely to resemble the in vivo environment, the cell structure preferably has a thickness of 5 $\mu$m or more, more preferably 30 $\mu$m or more, even more preferably 100 $\mu$m or more, and particularly preferably 150 $\mu$m or more. The thickness of the cell structure is preferably 500 $\mu$m or less, more preferably 400 $\mu$m or less, and even more preferably 200 $\mu$m or less. The upper and lower limits of the thickness of the cell structure can be combined as appropriate. For example, the thickness of the cell structure is preferably 5 $\mu$m or more and 500 $\mu$m or less, more preferably 30 $\mu$m or more and 400 $\mu$m or less, and may be 100 $\mu$m or more and 200 $\mu$m or less, or 150 $\mu$m or more and 200 $\mu$m or less.

(Method of producing second cell layer)

The second cell layer 140 (i.e., cell structure) containing cells forming the stroma can be any structure consisting of a single or multiple cell layers containing stromal cells, and the method of producing it is not particularly limited. For example, the structure including a plurality of cell layers may be produced by sequentially laminating cell layers containing stromal cells, by producing two or more cell layers at once, or by combining these methods as appropriate.

Moreover, the cell structure may be a multilayer structure in which the type of cell that constitutes each layer is different for each layer, or the type of cell that constitutes each layer may be the same in all the layers of the structure. For example, the cell structure may be produced by forming a layer for each cell type and sequentially laminating these cell layers. It is also possible to prepare a cell mixture containing a plurality of types of cells in advance, and use this cell mixture to produce a multilayer cell structure at once.

An example of the method of producing the cell structure by laminating each layer in sequence includes the method disclosed in JP 4919464 B, that is, repeating alternately a step of forming a cell layer and a step of bringing the formed cell layer into contact with a solution containing an extracellular matrix (ECM) component to laminate the cell layers in a continuous manner. For example, a cell mixture in which all the cells forming a cell structure are mixed is prepared beforehand, and then individual cell layers are formed using this cell mixture, thereby producing a cell structure in which a vascular network structure is formed over the entire cell structure. Further, by forming a cell layer for each cell type, a cell structure can be formed in which a vascular network structure is formed only in the layer consisting of endothelial cells.

As the method of producing two or more cell layers at once, for example, the method disclosed in JP 5850419 B can be used. According to the above method, the entire cell surface is previously coated with a polymer containing an arginine-glycine-aspartic acid (RGD) sequence to which integrins bind, and a polymer interacting with the polymer containing the RGD sequence. After the cells coated with the adhesive film are placed in a vessel, the coated cells are aggregated by centrifugation or the like to thereby produce a cell structure consisting of a plurality of cell layers. For example, the coated cells are prepared by previously preparing a cell mixture containing all cells that constitute a cell structure, and adding an adhesive component to the cell mixture. This enables the production of a cell structure having a uniform cell composition across its entirety by one centrifugation.

The second cell layer 140 (i.e., the cell structure) containing cells forming the stroma can be produced by a method including (a) obtaining a mixture containing cells including stromal cells, a cationic substance, an extracellular matrix component, and a polyelectrolyte; (b) causing the mixture to gel to obtain a gel composition; and (c) incubating the gel composition to obtain a cell structure. Each step will be described below.

First, in step (a), a mixture containing cells including the above-described stromal cells, a cationic substance, an extracellular matrix component, and a polyelectrolyte is obtained. The cells including stromal cells, cationic substance, extracellular matrix component, and polyelectrolyte may be mixed in an aqueous solvent. Examples of the aqueous solvent include water, buffers, and culture media.

<<Cationic substance>>

The cationic substance can be any substance having a positive charge can be used as long as it does not adversely affect cell growth. Examples of cationic substances include, but are not limited to, cationic buffers such as Tris-HCl, Tris-maleate, Bis-Tris, and HEPES, ethanolamine, diethanolamine, triethanolamine, polyvinylamine, polyallylamine, polylysine, polyhistidine, and polyarginine. Among these, cationic buffers are preferred, and Tris-HCl is more preferred.

The concentration of the cationic substance in the mixture in step (a) is not particularly limited as long as it does not adversely affect cell growth. The concentration of the cationic substance is preferably 10 to 100 mM, and may be, for example, 20 to 90 mM, 30 to 80 mM, 40 to 70 mM, or 45 to 60 mM.

When a cationic buffer is used as the cationic substance, the pH of the cationic buffer is not particularly limited as long as it does not adversely affect cell growth. The pH of the cationic buffer is preferably 6.0 to 8.0. For example, the pH of the cationic buffer may be 7.0, 7.1, 7.2, 7.3, 7.4, 7.5, 7.6, 7.7, 7.8, 7.9, or 8.0. The pH of the cationic buffer is more preferably in the range of 7.2 to 7.6, and even more preferably approximately 7.4.

<<Extracellular matrix component>>

The extracellular matrix component can be any component forming the extracellular matrix (also called ECM) as long as it does not adversely affect cell growth. Examples of the extracellular matrix component include, but are not limited to, collagen, laminin, fibronectin, vitronectin, elastin, tenascin, entactin, fibrillin, proteoglycan, and combinations thereof. Further examples of the extracellular matrix component include modifications and variants of the above examples. The extracellular matrix components may be used singly or in combination of two or more kinds.

Examples of the proteoglycan include chondroitin sulfate proteoglycan, heparan sulfate proteoglycan, keratan sulfate proteoglycan, and dermatan sulfate proteoglycan. Among these extracellular matrix components, collagen, laminin, and fibronectin are preferred, with collagen being particularly preferred.

The total content of the extracellular matrix component in the mixture in step (a) is not particularly limited as long as it does not adversely affect cell growth, and may be 0.005 mg/mL or more and 1.5 mg/mL or less, 0.005 mg/mL or more and 1.0 mg/mL or less, 0.01 mg/mL or more and 1.0 mg/mL or less, 0.025 mg/mL or more and 1.0 mg/mL or less, or 0.025 mg/mL or more and 0.1 mg/mL or less. The extracellular matrix component can be used by being dissolved in an appropriate solvent. Examples of appropriate solvents include, but are not limited to, water, buffers, and acetic acid. buffers and acetic acid are particularly preferred.

<<Polyelectrolyte>>

Polyelectrolyte refers to a polymer having a dissociable functional group in the polymer chain. Any polyelectrolyte can be used as long as it does not adversely affect cell growth. Examples of the polyelectrolyte include, but are not limited to, glycosaminoglycans such as heparin, chondroitin sulfate (e.g., chondroitin 4-sulfate and chondroitin 6-sulfate), heparan sulfate, dermatan sulfate, keratan sulfate, and hyaluronic acid; dextran sulfate, rhamnan sulfate, fucoidan, carrageenan, polystyrene sulfonic acid, polyacrylamide-2-methylpropanesulfonic acid, polyacrylic acid, and combinations thereof. Further examples of the polyelectrolyte are derivatives of the above examples. These polyelectrolytes may be used singly or in combination of two or more.

The polyelectrolyte is preferably a glycosaminoglycan. Among glycosaminoglycans, heparin, chondroitin sulfate, and dermatan sulfate are preferred, with heparin being particularly preferred.

The concentration of the polyelectrolyte in the mixture in step (a) is not particularly limited as long as it does not adversely affect cell growth. Unlike the extracellular matrix component, at any concentration lower than or equal to solubility limit, the polyelectrolyte can be effective and do not inhibit the effects of the extracellular matrix component. The concentration of the polymer electrolyte is preferably 0.005 mg/mL or more, and may be 0.005 mg/mL or more and 1.0 mg/mL or less, 0.01 mg/mL or more and 1.0 mg/mL or less, 0.025 mg/mL or more and 1.0 mg/mL or less, or 0.025 mg/mL or more and 0.1 mg/mL or less.

The polyelectrolyte can be used by being dissolved in an appropriate solvent. Examples of appropriate solvents include, but are not limited to, water and buffers. When a cationic buffer is used as the cationic substance, the polyelectrolyte may be dissolved in the cationic buffer.

The composition ratio (final concentration ratio) of the polyelectrolyte to the extracellular matrix component in the mixture in step (a) is preferably 1:2 to 2:1, may be 1:1.5 to 1.5:1, or may be 1:1.

In step (a), the cells including the above-described stromal cells, the cationic substance, the extracellular matrix component, and the polyelectrolyte can be mixed in a suitable vessel such as a dish, tube, flask, bottle, well plate, or cell culture insert. The mixing may also be carried out in a vessel used in step (b).

The mixture obtained in step (a) may contain other components in addition to the cells including the above-described stromal cells, the cationic substance, the extracellular matrix component, and the polyelectrolyte. Examples of the other components include a gelling agent required to obtain a gel composition in step (b), and a cell culture medium.

<<Gelling agent>>

Examples of gelling agents include extracellular matrix components, agarose, pectin, and combinations of fibrinogen and thrombin. The gelling agent may be contained in advance in the mixture in step (a), or may be added to the mixture obtained in step (a) in step (b) described below.

In step (b) following step (a), the mixture obtained in step (a) is caused to gel to obtain a gel composition. The gelling method varies depending on the gelling agent used. For example, it may be carried out by placing the mixture obtained in step (a) under gelling conditions. Alternatively, a gelling agent may be added to the mixture obtained in step (a) before placing the mixture under gelling conditions.

For example, when an extracellular matrix component is used as the gelling agent, the gelling conditions include, for example, allowing the mixture obtained in step (a) to stand at approximately 37°C. This causes the extracellular matrix component contained in the mixture in step (a) to gel and produced a gel composition. Alternatively, an extracellular matrix component may be further added to the mixture obtained in step (a) before being allowed to stand at approximately 37°C to cause gelation.

When agarose is used as the gelling agent, it may be added to the mixture obtained in step (a), dissolved at a temperature equal to or higher than the melting point of the agarose used, and then allowed to stand at a temperature equal to or lower than the solidifying point of the agarose used to cause gelation.

When pectin is used as the gelling agent, it may be added to the mixture obtained in step (a). The pectin gels due to divalent ions such as calcium ions contained in the mixture and produces a gel composition.

It is also possible to use fibrinogen and thrombin may as gelling agents. Thrombin, which is a serine protease, cleaves fibrinogen into fibrin monomers. Calcium ions cause fibrin monomers to polymerize with each other to form poorly soluble fibrin polymer. In vivo, fibrin polymers are cross-linked by the action of factor XIII, a fibrin stabilizing factor, to form mesh-like fibers called stabilized fibrin that cause blood coagulation. The gel composition obtained by gelation of fibrin polymers may be referred to as fibrin gel herein.

That is, step (b) of obtaining a gel composition may include mixing thrombin and fibrinogen with the mixture obtained in step (a). The gel composition obtained in step (b) may contain fibrin gel as a gel component.

In that case, it is preferable that step (b) of obtaining a gel composition includes step (b1) of adding thrombin to the mixture obtained in step (a), and step (b2) of adding fibrinogen to the mixture to which thrombin has been added so that a fibrin gel is formed and the mixture gels.

When fibrinogen and thrombin are used as gelling agents, the concentration of fibrinogen in the mixture in step (b) is preferably 0.5 mg/mL or more and 25 mg/mL or less. A concentration of 0.5 mg/mL or more facilitates gelation when the fibrinogen is mixed with thrombin. A concentration of 25 mg/mL or less helps the fibrinogen to dissolve in the mixture. The thrombin is preferably dissolved or dispersed in the mixture in step (b).

Next, in step (c), the gel composition obtained in step (b) is incubated to obtain the cell structure. The incubation of the gel composition for obtaining the cell structure may be carried out for 5 minutes to 72 hours. Step (c) has the effect of promoting adhesion between the cells contained in the gel composition so that a stable cell structure is obtained.

The vessel used in step (c) may be the same as that used in step (b). In step (c), the vessel used in step (b) may be directly used, or the contents may be transferred to another vessel.

The cell culture in step (c) can be carried out under culture conditions suitable for the cells to be cultured. Those skilled in the art can select an appropriate medium depending on the cell type and the desired function. The medium is not particularly limited, and examples thereof include media obtained by adding approximately 1 to 20 vol% serum to a basal medium such as DMEM, EMEM, MEMα, RPMI-1640, McCoy's 5A, or Ham's F-12. Examples of the serum include calf serum (CS), fetal bovine serum (FBS), and fetal horse serum (HBS). When the stromal cells include vascular endothelial cells, a growth factor such as VEGF, EGF, or FGF may be added to the medium. Various conditions of the culture environment, such as temperature and atmospheric composition, may be adjusted to be suitable for the cells to be cultured.

Step (c) may be carried out after steps (a) and (b) have been carried out two or more times. A cell structure having multiple layers can be produced by repeating steps (a) and (b). In other words, a thick cell structure can be produced.

Alternatively, steps (a) and (b) can be repeated, using a different cell population for each iteration, to produce cell structures composed of different types of cells.

Fig. 2 is a schematic cross-sectional view illustrating an example of a method of producing the second cell layer (i.e., the cell structure) containing cells that form the stroma. First, the cylindrical member 150 is placed inside a suitable vessel 210. Here, one opening 151 of the cylindrical member is brought into close contact with a bottom surface 211 of the vessel 210. The vessel 210 is not particularly limited. For example, it may be a cell culture dish.

Next, the mixture obtained in step (a) is placed inside the cylindrical member 150, and step (b) is carried out. The mixture gels and produces a gel composition 140'. Since one of opening 151 of the cylindrical member 150 is in close contact with the bottom surface 211 of the vessel 210, the mixture does not leak from the interface between the cylindrical member 150 and the bottom surface 211.

After that, step (c) is carried out to promote adhesion between the cells contained in the gel composition 140' so that a stable cell structure is obtained, thereby producing the second cell layer 140 (i.e., the cell structure) containing cells that form the stroma.

As mentioned above, a membrane may be provided in one of opening 151 of the cylindrical member. The membrane may or may not be removed after the second cell layer 140 is formed.

Since the second cell layer 140 is gelled, it can be easily peeled off from the bottom surface 211 of the vessel 210. The second cell layer 140 thus obtained can be placed on the first cell layer 130 containing the primary cells 131 located on the cell culture surface 111 of the cell culture vessel 110. As a result, the primary cells are brought into close contact with the cell structure containing stromal cells, making it possible to culture the primary cells.

As mentioned above, a membrane may be provided in one of opening 151 of the cylindrical member. In that case, the primary cells are in contact with the cell structure containing stromal cells via the membrane.

The second cell layer 140 may be placed on the first cell layer 130 together with the cylindrical member 150 as shown in Fig. 1, or the second cell layer 140 may be removed from the cylindrical member 150 and then placed on the first cell layer 130.

The second cell layer 140 can be easily detached from the first cell layer 130 upon subculture, which will be described later. In other words, the second cell layer 140 is removable from the first cell layer 130. In particular, when the second cell layer 140 is gelled, the first and second cell layers 130 and 140 can be easily separated from each other.

[Cell culture method]

In one embodiment, the present invention provides a method for culturing cells that includes incubating the cell-containing vessel described above, and exchanging the second cell layer to subculture the cells contained in the first cell layer.

More specifically, the culture method of this embodiment includes seeding first cells in a cell culture vessel to obtain the first cell layer located on the cell culture surface of the cell culture vessel, placing the second cell layer containing second cells forming the stroma on the first cell layer to obtain a cell-containing vessel including the cell culture vessel, the cell culture medium, and the first and second cell layers, incubating the cell-containing vessel, and subculturing the first cells, in which the second cell layer is replaced with a new one during the subculturing of the first cells.

In the culture method of this embodiment, the cell culture vessel, the cell culture medium, the cells, the first cell layer, the cells forming the stroma, the second cell layer, and the like are the same as those described above.

As will be described later using Examples, according to the cell culture method of this embodiment, it is possible to culture primary cells or cells other than primary cells using a general cell culture medium without adding growth factors or inhibitors of signal transduction pathways. In addition, as described above, the first and second cell layers can be easily separated. This facilitates exchange of the second cell layer, and the first cells can be cultured for a long period of time even when they are primary cells.

In the culture method of this embodiment, the frequency of exchange of the second cell layer may be adjusted as appropriate depending on the cells being cultured. When the cells are primary cells, the second cell layer may be exchanged, for example, every 2 to 10 days.

[Method for evaluating the effect of drug on cells]

In one embodiment, the present invention provides a method for evaluating an effect of a drug on cells. The method includes incubating the cell-containing vessel described above in the presence of the drug, exchanging the second cell layer to subculture the cells contained in the first cell layer, and evaluating the effect of the drug on the cells contained in the first cell layer.

As will be described later using Examples, according to the cell culture method of this embodiment, it is possible to culture primary cells or cells other than primary cells using a general cell culture medium without adding growth factors or inhibitors of signal transduction pathways. Therefore, in particular, when the cells contained in the first cell layer are primary cells, it is considered that the behavior of the cells can be evaluated under conditions closer to those in vivo. In addition, since the second cell layer can be easily exchanged and the cells can be cultured for a long period of time, the evaluation is less affected by external environmental factors and the cells can be cultured and evaluated under more stable conditions.

Furthermore, since the second cell layer can be easily exchanged, the time and work required for subculturing the cells contained in the first cell layer can be significantly reduced. This provides advantageous effects including less contamination during subculturing, subculturing with a high cell recovery rate, and a small loss of cells.

The drug is not particularly limited, and, for example, it may be an anti-cancer drug when the cells contained in the first cell layer are cancer cells. Alternatively, the method of this embodiment can be said to be a drug screening method. In that case, the drug may be, for example, a natural compound library, a synthetic compound library, or an existing drug library. This screening method enables screening drugs using cells. In the method of this embodiment, the cells contained in the first cell layer may be primary cells or cells other than primary cells.

Examples

The present invention will be described in more detail referring to the examples described below, but the present invention is not limited to the examples.

[Materials and methods]

(Stroma-forming celles)

The cells shown in Table 1 below were used as the stroma-forming celles.

[Table 1]

| Cell name | Origin | Manufacturer | Product No. |
|-----------|--------|--------------|-------------|
| NHDF | Human neonatal dermal fibroblasts | Lonza | CC-2509 |
| HUVEC | Human umbilical vein endothelial cells | Lonza | CC-2517A |

(Reagents)

Table 2 below shows the reagents used in the experimental examples described later.

[Table 2]

| Product name | Manufacturer | Product No. |
|---|---|---|
| Collagen | Sigma | ASC-1-100-100 |
| Heparin | Sigma | H3149-100KU |
| Tris HCl | FUJIFILM Wako Pure Chemical | 166-23555 |
| Fibrinogen | Sigma | F8630-5G |
| Thrombin | Sigma | T4648-10KU |
| DMEM | FUJIFILM Wako Pure Chemical | 043-30085 |
| EGM-2MV | Lonza | CC-3202 |
| FBS | Thermo | P0241817 |
| Trypsin | Invitrogen | 25200072 |
| Formalin buffer | FUJIFILM Wako Pure Chemical | 062-01661 |
| PBS | FUJIFILM Wako Pure Chemical | 166-23555 |
| StemPro™ hESC SFM | Gibco | A1000701 |
| BSA | Gibco | A10008-011 |
| Recombinant Human FGF-Basic | Thermo | PHG0266 |
| 2-Mercaptoethanol | Invitrogen | #21985-023 |
| Antibiotic-Antimycotic | Gibco | 15240062 |
| DMEM/F-12, GlutaMAX | Lonza | 12-168F |

(Cell culture vessel)

Table 3 below shows the cell culture vessels used in the experimental examples described later.

[Table 3]

| Name | Product name | Manufacturer | Product No. |
|---|---|---|---|
| First vessel (cylindrical member) | Transwell culture insert | Corning | 3470 |
| Second vessel | Petri dish | Iwaki | - |
| Third vessel | 24-well plate | Corning | 3526 |

(Cell culture medium)

Table 4 below shows the cell culture media used in the experimental examples described later. In the following, the cell layer containing stroma-forming celles, that is, the second cell layer, may be referred to as "stromal stamp".

[Table 4]

| Name | Composition |
|---|---|
| General-purpose medium | 10% FBS<br>1% Antibiotics (penicillin/streptomycin)<br>DMEM |

(continued)

| Name | Composition |
|---|---|
| Primary culture medium | 50% StemPro™ hESC SFM<br>1.8% BSA<br>8.0 ng/mL Recombinant Human FGF-Basic<br>0.1 mM 2-Mercaptoethanol<br>10 µM Y-276321<br>1% Antibiotic-Antimycotic<br>GlutaMAX™<br>DMEM/F-12 |
| Vascular cell medium | EGM-2MV medium |
| Stromal stamp medium | Culture obtained by mixing general-purpose medium and vascular cell medium at 1:1 |

[Experimental Example 1]

(Preparation of stromal stamp)

A cell layer containing stroma-forming celles (i.e., the stromal stamp) was prepared as follows. First, a 10 U/mL thrombin-DMEM solution was prepared. A 10 mg/mL fibrinogen-DMEM solution was also prepared.

$1 \times 10^6$ or more NHDFs and HUVECs, at 1/10 to 1/5 the number of the NHDFs, were suspended in a 50 mM Tris-HCl buffer (pH 7.4) containing 0.05 mg/mL heparin and 0.05 mg/mL collagen.

Subsequently, the mixture was centrifuged at $1000 \times g$ for 1 minute at room temperature, and the supernatant was removed. The precipitate was then suspended in a general-purpose medium to obtain a suspension. The suspension was mixed with the fibrinogen solution at a ratio of 1:1 (v/v). The mixture was mixed with the thrombin solution at a ratio of 2:1 (v/v).

The membrane of a first vessel (Transwell culture insert) in Table 3 above was removed to obtain a cylindrical member having openings at both ends. The first vessel was placed on a second vessel (Petri dish) of Table 3 above, and 100 µL of the above suspension was seeded inside the first vessel. The suspension was allowed to stand in a $CO_2$ incubator (37°C, 5% $CO_2$) until it gelled.

Then, an appropriate amount of medium for the stromal stamp was added inside the first vessel and into the second vessel, and culturing was performed for 3 days inside a $CO_2$ incubator (37°C, 5% $CO_2$). After that, the first vessel was removed from the second vessel. The stromal stamp was thus obtained having a structure in which one of the two end openings of the cylindrical member was blocked with gel.

[Experimental Example 2]

(Culture of primary cells)

The stromal stamp was used to culture primary cells. Primary cells were also cultured using a conventional 2D culture method and a conventional 3D culture method for comparison.

<<Preparation of primary cells>>

Commercially available fresh frozen breast cancer tissue (Proteogenomics, 009-01310) and fresh frozen pancreatic cancer tissue (Proteogenomics, 009-01310) were used as the primary cells. After treatment with collagenase/dispase, each tissue was passed through a filter with a pore size of 100 µm, and the fraction that passed was used as primary cells.

<<Primary cell culture using stromal stamp>>

The primary cells were seeded at $2 \times 10^4$ cells/well in a third vessel (24-well plate) shown in Table 3 above. The stromal stamp prepared in Example 1 was then placed on each well. As a result, the stromal stamp (i.e., the second cell layer containing cells forming the stroma) was placed in contact with the first cell layer containing primary cells. The cells were then cultured in the general-purpose medium shown in Table 4 above (N = 3).

On days 2, 7, 14, 21, and 28 after the start of culture, the stromal stamps were removed and the number of primary cells was counted by ATP assay. After counting the cells, a new stromal stamp was placed on each well. The proliferation rate of the primary cells was calculated using the following formula (1).

$$\text{Proliferation rate (fold)} = \text{cell count} / \text{number of cells seeded} \ldots (1)$$

<<Primary cell culture using 2D culture method>>

The primary cells were seeded at $2 \times 10^4$ cells/well in a third vessel (24-well plate) shown in Table 3 above. The cells were then cultured in the general-purpose medium or the medium for primary culture shown in Table 4 above (N = 3). On days 2, 7, 14, 21, and 28 from the start of culture, the number of primary cells was counted by ATP assay. The proliferation rate of the primary cells was calculated using the above formula (1).

<<Primary cell culture using 3D culture method>>

$2.0 \times 10^6$ NHDFs and $3.0 \times 10^4$ HUVECs were suspended in an equal volume mixture of 150 $\mu$L of 0.2 mg/mL heparin/50 mM Tris-HCl buffer (pH 7.4) and 150 $\mu$L of 0.2 mg/mL collagen/5 mM acetate solution (pH 3.7). The resulting mixture was centrifuged at $1,000 \times$ g for 1 minute at room temperature to obtain a viscous mass. The obtained viscous mass was suspended in DMEM containing 10% FBS. The entire suspension was seeded in a 24-well Transwell culture insert and centrifuged at $400 \times$ g (gravitational acceleration) at room temperature for 1 minute. As a result, a cell layer (i.e., a cell layer containing cells forming the stroma) was formed on the membrane of the Transwell culture insert.
Subsequently, primary cells were seeded on the cell layer at $2 \times 10^4$ cells/well and cultured in the general-purpose medium shown in Table 4 above (N = 3). On days 2, 7, 14, 21, and 28 after the start of culture, the cells were fixed and stained for EpCAM with immunofluorescence staining, and the stained cells were counted as primary cells. The proliferation rate of the primary cells was calculated using the above formula (1).
Table 5 below shows the results of culturing primary cell derived from fresh frozen breast cancer tissue. The results of 2D culture show that primary cells derived from fresh frozen breast cancer tissue did not proliferate in either the culture using the general-purpose medium or the culture using the primary culture medium. As for 3D culture, proliferation of primary cells was confirmed up to 14 days after the start of culture, but proliferation stopped after 14 days. In contrast, in the case of culture using the stromal stamp, proliferation of primary cells was confirmed over a period of 21 days after the start of culture, and had the highest proliferation efficiency.

[Table 5]

| Culture days | | 2 | 7 | 14 | 21 |
|---|---|---|---|---|---|
| Proliferation | 2D culture | 0.7±0.32 | 0±0 | 0±0 | 0±0 |
| rate (fold) | General-purpose medium | | | | |
| | 2D culture Primary culture medium | 0.76±0.06 | 1.3±0. 23 | 0.4±0.54 | 0.2±0.32 |
| | 3D culture General-purpose medium | 0.6±0.06 | 1.7±0.34 | 2.4±0.46 | 2.3±0.35 |
| | Stromal stamp General-purpose medium | 0.8±0.04 | 1.6±0.33 | 2±0.65 | 3.4±0.56 |

Table 6 below shows the results of culturing primary cell derived from fresh frozen pancreatic cancer tissue. No proliferation of primary cells derived from fresh frozen pancreatic cancer tissue was observed when 2D culture was carried out using the general-purpose medium. When 2D culture was carried out using the primary culture medium, the primary cells tended to proliferate slowly up to 21 days after the start of culture, but decreased after that. As for 3D culture, proliferation of primary cells was confirmed up to 21 days after the start of culture, but proliferation stopped after that. In contrast, in the case of culture using the stromal stamp, proliferation of primary cells was confirmed over a period of 28 days after the start of culture, and had the highest proliferation efficiency.

[Table 6]

| Culture days | | 7 | 14 | 21 | 28 |
|---|---|---|---|---|---|
| Proliferation rate (fold) | 2D culture General-purpose medium | 0.1±0.23 | 0±0 | 0±0 | 0±0 |
| | 2D culture Primary culture medium | 0.66±0.1 | 0.62±0.08 | 0.93±0.12 | 0.45±0.12 |
| | 3D culture General-purpose medium | 0.76±0.13 | 1.23±0. 23 | 1.56±0. 34 | 1.53±1.01 |
| | Stromal stamp General-purpose medium | 1.1±0.13 | 1.43±0. 21 | 2.14±0. 86 | 5.23±1.21 |

[Experimental Example 3]

(Evaluation of anti-cancer drug)

An anti-cancer drug was evaluated using cancer cells cultured using the stromal stamp. In addition, for comparison, cancer cells were cultured using a conventional 2D culture method and a conventional 3D culture method, and the anti-cancer drug was evaluated using the cultured cells.

<<Cancer cell culture and evaluation of anti-cancer drug using stromal stamp>>

HT29 (ATCC, HTB-38), a human colon adenocarcinoma cell line, was seeded in the third vessel (24-well plate) in Table 3 above at $1 \times 10^3$ cells/well. The stromal stamp prepared in Example 1 was then placed on each well. The cells were then cultured in the general-purpose medium shown in Table 4 above.
On the fourth day after the start of the culture, an anti-cancer drug SN-38 (Selleck, S4908) was added to the medium in each well at a concentration of 0, 0.01, 0.1, 1 or 10 $\mu$M. On the seventh day after the start of culture, the stromal stamps were removed and the number of HT29 cells was counted by ATP assay. The cell viability was calculated using the following formula (2) to evaluate the drug efficacy.

Cell viability (%) = number of cells in the presence of drug at each concentration / number of cells in the absence of drug $\times$ 100 (2)

<<Cancer cell culture and evaluation of anti-cancer drug using 2D culture method>>

The HT29 cells were seeded at $1 \times 10^3$ cells/well in the third vessel (24-well plate) shown in Table 3 above. The cells were then cultured in the general-purpose medium shown in Table 4 above.
On the fourth day after the start of the culture, an anti-cancer drug SN-38 (Selleck, S4908) was added to the medium in each well at a concentration of 0, 0.01, 0.1, 1 or 10 $\mu$M. On the seventh day after the start of culture, the number of HT29 cells was counted by ATP assay. The cell viability was calculated using the above formula (2) to evaluate the drug efficacy.

<<Cancer cell culture and evaluation of anti-cancer drug using 3D culture method>>

$2.0 \times 10^6$ NHDFs and $3.0 \times 10^4$ HUVECs were suspended in an equal volume mixture of 150 $\mu$L of 0.2 mg/mL heparin/50 mM Tris-HCl buffer (pH 7.4) and 150 $\mu$L of 0.2 mg/mL collagen/5 mM acetate solution (pH 3.7). The resulting mixture was centrifuged at 1,000 $\times$ g for 1 minute at room temperature to obtain a viscous mass. The obtained viscous mass was suspended in DMEM containing 10% FBS. The entire suspension was seeded in a 24-well Transwell culture insert and centrifuged at 400 $\times$ g (gravitational acceleration) at room temperature for 1 minute. As a result, a cell layer (i.e., a cell layer containing cells forming the stroma) was formed on the membrane of the Transwell culture insert.
Subsequently, HT29 cells were seeded on the cell layer at $1 \times 10^3$ cells/well and cultured in the general-purpose medium shown in Table 4 above. On the fourth day after the start of the culture, an anti-cancer drug SN-38 (Selleck, S4908) was added to the medium in each well at a concentration of 0, 0.01, 0.1, 1 or 10 $\mu$M.
On the seventh day after the start of culture, the cells were fixed and stained for EpCAM with immunofluorescence staining, and the stained cells were counted as HT29 cells. More specifically, the area of fluorescence emission from cancer cells was calculated using a microscope system (Operetta CLS, PerkinElmer) in the fluorescence mode (fluorescence: Alexa 647). The cell viability was calculated using the following formula (3) to evaluate the drug efficacy.

Cell viability (%) = area of fluorescence emission from cancer cells in the presence of drug at each concentration/area of fluorescence emission from cancer cells in the absence of drug $\times$ 100 (3)

The evaluation results of the anti-cancer drug are shown in Table 7 below. The cell viability tended to decrease in a drug concentration-dependent manner regardless of whether the culture was performed using the stromal stamp, the 2D culture method, or the 3D culture method. In the case where the stromal stamp was used, drug resistance was higher than when the 2D culture method was used, and evaluation results were substantially equivalent to those when the 3D culture method was used. This demonstrated that a simple evaluation method using the stromal stamp can achieve anti-cancer drug evaluation substantially equivalent to that achieved by the 3D culture method.

[Table 7]

| Concentration($\mu$M) | | 0.01 | 0.1 | 1 | 10 |
|---|---|---|---|---|---|
| Cell viability (%) | 2D culture | 78.3±9.45 | 54.3±5. 45 | 35±3. 24 | 21.3±4. 23 |
| | 3D culture | 97.8±20.04 | 72.87±14.57 | 54.21±13.05 | 34.2±9.47 |
| | Stromal stamp | 103.3±14.5 | 72.87±14.57 | 49.3±13.05 | 36.5±10. 24 |

[Experimental Example 4]

(Culture of fertilized mouse eggs)

The stromal stamp was used to culture fertilized mouse eggs. In addition, fertilized mouse eggs were cultured using the conventional 2D culture method in a specialized medium and the conventional 2D culture method in a general-purpose medium for comparison.

<<Culture of fertilized mouse eggs in specialized medium using 2D culture method>>

Fertilized eggs (C57BL/6J Jcl, ARK resource) were transferred to a drop of 100 $\mu$L of fertilized mouse egg-specific medium (KSOM/AA medium, ARK resource) or 100 $\mu$L of general-purpose medium applied to a 35 mm culture dish (430165, Corning), and cultured for 4 days under conditions of 37°C, 5% $O_2$, and 5% $CO_2$ (N = 10). The embryos on the fourth day of culture were observed under a microscope, and the proportion of embryos that had reached the blastocyst stage from fertilized eggs was calculated.

<<Culture of fertilized mouse eggs in general-purpose medium using 2D culture method>>

The experiment was performed in the same manner as in "Culture of fertilized mouse eggs in specialized medium using 2D culture method", except that the general-purpose medium shown in Table 4 was used instead of the fertilized mouse egg-specific medium. The embryos on the fourth day of culture were observed under a microscope, and the proportion of embryos that had reached the blastocyst stage from fertilized eggs was calculated.

<<Culture 1 of fertilized mouse eggs using stromal stamp>>

Fertilized eggs (C57BL/6J Jcl, ARK resource) were transferred to a drop of 100 $\mu$L of a combined solution of 10 U/mL thrombin-DMEM solution and 10 mg/mL fibrinogen-DMEM solution applied to a 35 mm culture dish (430165, Corning), and left to stand until gelation occurred and a drop gel was obtained. After the gelation, the stromal stamp prepared in Example 1 was placed on each drop gel. They were cultured in the general-purpose medium shown in Table 4 for 4 days under conditions of 37°C, 5% $O_2$ and 5% $CO_2$ (N = 10). The embryos on the fourth day of culture were observed under a microscope, and the proportion of embryos that had reached the blastocyst stage from fertilized eggs was calculated.

<<Culture 2 of fertilized mouse eggs using stromal stamp>>

An experiment was performed in the same manner as in "Culture 1 of fertilized mouse eggs using stromal stamp" except that, instead of the stromal stamp prepared in Experimental Example 1, a stromal stamp for fertilized egg culture was used, prepared by changing the cell composition so that VECs (mouse vaginal epithelial cells, established by TOPPAN Holdings Inc.) and HUVECs are used instead of NHDFs. The embryos on the fourth day of culture were observed under a microscope, and the proportion of embryos that had reached the blastocyst stage from fertilized eggs was calculated.

Table 8 below shows the results of the culture evaluation of fertilized mouse eggs. When the general-purpose medium was used, no embryos developed to the blastocyst stage in 2D culture. On the other hand, in the case where the stromal stamp was used, the proportion of embryos that reached the blastocyst stage was 60% when the stromal stamp used in Experimental Example 1 was used, and 80% when the stromal stamp prepared with the cell composition for fertilized egg culture was used, which is the same as when the fertilized egg-specific medium was used. This shows that by using the stromal stamp, even when a general-purpose medium is used, a blastocyst development rate can be achieved that is substantially the same as that achieved by the fertilized egg-specific medium.

[Table 8]

| | 2D culture Fertilized egg-specific medium | 2D culture General-purpose medium | Stromal stamp of Experimental Ex. 1 General-purpose medium | Stromal stamp for fertilized egg culture General-purpose medium |
|---|---|---|---|---|
| Blastocyst development rate (%) | 80 | 0 | 60 | 80 |

[Experimental Example 5]

(IPS cell differentiation)

The stromal stamp was used to induce differentiation of IPS cells. IPS cells were also cultured using a conventional 2D culture method for comparison.

<<Induction of differentiation of IPS cells into cardiac muscle cells by 2D culture method using specialized medium>>

According to the method described in JP 6,429,280 B (Additional Comparative Example 1), naive IPS cells (prepared using the AlphaSTEM (registered trademark) culture system) were induced to differentiate into cardiac muscle cells using a medium designed for inducing the differentiation, and the number of days required for the proportion of cTNT positive cells to reach 80% or more was compared. The evaluation was outsourced to Fukushima Cell Factory.

<<Induction of differentiation of IPS cells into cardiac muscle cells by 2D culture method using general-purpose medium>>

An experiment was performed according to the method described in "Induction of differentiation of IPS cells into cardiac muscle cells by 2D culture method using specialized medium", except that the general-purpose medium shown in Table 4 was used instead of the medium designed for inducing cardiac differentiation described in JP 6,429,280 B. The number of days required for the proportion of cTNT positive cells to reach 80% or more was compared. The evaluation was outsourced to Fukushima Cell Factory.

<<Induction of differentiation of IPS cells into cardiac muscle cells using stromal stamp>>

Naive IPS cells (prepared by a contractor using the AlphaSTEM (registered trademark) culture system) were transferred to a drop of 200 $\mu$L of a combined solution of 10 U/mL thrombin-DMEM solution and 10 mg/mL fibrinogen-DMEM solution to produce a drop gel. An early differentiation-stage stromal stamp was prepared in the same manner as in Experimental Example 1, except that the cellular composition was changed so that NHDFs were replaced by GFP-introduced human cardiac fibroblasts (HCF, D10057, manufactured by PROMOCELL), and HUVECs were replaced by YFP-introduced human cardiac myocytes (HCM, D10115, manufactured by PROMOCELL), human mesenchymal stem cells from umbilical cord matrix (hMSC-UC, product number: D10130, manufactured by PROMOCELL), and human mesenchymal stem cells from adipose tissue (hMSC-AT, product number D10134, manufactured by PROMOCELL). The early differentiation-stage stromal stamp was placed on each drop gel. Then, they were induced to differentiate in the general-purpose medium for 2 days under conditions of 37°C, 5% $O_2$ and 5% $CO_2$. Next, a stromal stamp for maintaining stability was prepared using the method described in Experimental Example 1, except that the cell composition was changed to GFP-introduced human cardiac fibroblasts (HCF, product number: D10057, manufactured by PROMOCELL) and YFP-introduced human cardiac myocytes (HCM, product number: D10115, manufactured by PROMOCELL). After removing the early differentiation-stage stromal stamp from each drop gel, the stromal stamp for maintaining stability was placed on each drop gel to induce and maintain differentiation, and the number of days required for the proportion of cTNT positive cells to reach 80% or more was compared. The evaluation was outsourced to Fukushima Cell Factory.

The evaluation results of induction of cardiac differentiation are shown in Table 9 below. In the cases where the general-purpose medium was used, when the 2D culture method was used, no signs of differentiation were observed and the cells could not be maintained. On the other hand, when the stromal stamp was used, the proportion of cTNT positive cells reached 80% or more after $15 \pm 3$ days. Furthermore, when the stromal stamp was used, the number of days required for the proportion of cTNT positive cells to reach 80% or more was slightly less than that when the 2D culture method was used with a medium designed for inducing cardiac differentiation. This shows that by using the stromal stamp, even when a general-purpose medium is used, the number of days required for the proportion of cTNT positive cells to reach 80% or more was equivalent to or better than that when a conventional method is used.

[Table 9]

|  | 2D culture Inducing cardiac differentiation-specific medium | 2D culture General-purpose medium | Stromal stamp General-purpose medium |
|---|---|---|---|
| The number of days required for the proportion of cTNT positive cells to reach 80% or more (days) | $21 \pm 4$ | Not reached | $15 \pm 3$ |

[Industrial Applicability]

According to the present invention, it is possible to provide a technique for long-term culture of primary cells using a general cell culture medium without adding growth factors or inhibitors. The present invention can also be applied to cells other than primary cells.

[Reference Signs List]

| 100 | Cell-containing vessel |
|---|---|
| 110 | Cell culture vessel |
| 111 | Cell culture surface |
| 120 | Cell culture medium |
| 130 | First cell layer |
| 131 | Cell |
| 140 | Second cell layer (cell structure) |
| 140' | Gel composition |
| 151, 152 | Opening |
| 150 | Cylindrical member |
| 210 | Vessel |
| 211 | Bottom surface |

**Claims**

1.   A cell-containing vessel comprising:

a cell culture vessel;
a cell culture medium contained in the cell culture vessel;
a first cell layer located on a cell culture surface of the cell culture vessel; and
a second cell layer that contains stroma-forming celles and is located on the first cell layer, the second cell layer being removable from the first cell layer.

2.   The cell-containing vessel according to claim 1, wherein the first cell layer contains primary cells.

3.   The cell-containing vessel according to claim 1, further comprising a cylindrical member having openings at both ends,

wherein the cylindrical member is contained in the cell culture vessel so that one of the openings is in contact with the first cell layer, and
the second cell layer is placed inside the cylindrical member.

4. The cell-containing vessel according to any one of claims 1 to 3, wherein the second cell layer has been gelled.

5. A method for culturing cells, comprising:

incubating the cell-containing vessel according to any one of claims 1 to 3, and
exchanging the second cell layer to subculture cells contained in the first cell layer.

6. A method for evaluating an effect of a drug on a cell, the method comprising:

incubating the cell-containing vessel according to any one of claims 1 to 3 in the presence of the drug,
exchanging the second cell layer to subculture cells contained in the first cell layer, and
evaluating the effect of the drug on the cells contained in the first cell layer.

FIG.1

FIG.2

## INTERNATIONAL SEARCH REPORT

International application No.

**PCT/JP2024/001432**

| A. | CLASSIFICATION OF SUBJECT MATTER |
|---|---|

*C12M 3/00*(2006.01)i; *C12N 5/071*(2010.01)i; *C12N 5/09*(2010.01)i; *C12Q 1/06*(2006.01)i
FI:  C12M3/00 A; C12N5/071; C12N5/09; C12Q1/06

According to International Patent Classification (IPC) or to both national classification and IPC

| B. | FIELDS SEARCHED |
|---|---|

Minimum documentation searched (classification system followed by classification symbols)

C12M3/00; C12N5/071; C12N5/09; C12Q1/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed

| C. | DOCUMENTS CONSIDERED TO BE RELEVANT |
|---|---|

| Category* | Citation of document, with indication, where appropriate, of the relevant passages | Relevant to claim No. |
|---|---|---|
| X | HOPKINS, Timothy et al. An In Vitro System to Study the Effect of Subchondral Bone Health on Articular Cartilage Repair in Humans. Cells. 27 July 2021, vol. 10, issue 8, article no. 1903, pp. 1-27, DOI: 10.3390/cells10081903<br>Materials and Methods, fig. 3 | 1-5 |
| Y | Materials and Methods, fig. 3 | 6 |
| Y | WO 2021/075528 A1 (PUBLIC UNIVERSITY CORPORATION YOKOHAMA CITY UNIVERSITY) 22 April 2021 (2021-04-22)<br>claims | 6 |
| Y | WO 2007/072953 A1 (PHARMACO CELL COMPANY LTD.) 28 June 2007 (2007-06-28)<br>claims | 6 |
| Y | JP 2021-048834 A (KYOTO UNIVERSITY) 01 April 2021 (2021-04-01)<br>claims | 6 |

☐ Further documents are listed in the continuation of Box C.          ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **28 February 2024** | **19 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

International application No.

**PCT/JP2024/001432**

| Patent document cited in search report | | | Publication date (day/month/year) | Patent family member(s) | | | Publication date (day/month/year) |
|---|---|---|---|---|---|---|---|
| WO | 2021/075528 | A1 | 22 April 2021 | EP claims | 4047082 | A1 | |
| | | | | CN | 114585746 | A | |
| | | | | KR | 10-2022-0083699 | A | |
| WO | 2007/072953 | A1 | 28 June 2007 | US claims | 2010/0273200 | A1 | |
| | | | | EP | 1964915 | A1 | |
| JP | 2021-048834 | A | 01 April 2021 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023008762 A
- JP 5652809 B
- WO 2019039457 A
- JP 4919464 B
- JP 5850419 B
- JP 6429280 B

**Non-patent literature cited in the description**

- **TAKAMURA Y. et al.** Prediction of chemotherapeutic response by collagen gel droplet embedded culture-drug sensitivity test in human breast cancers. *Int. J. Cancer*, 2002, vol. 98, 450-455
- **ZHANG L. et al.** ROCK Inhibitor Y-27632 Suppresses Dissociation-Induced Apoptosis of Murine Prostate Stem/Progenitor Cells and Increases Their Cloning Efficiency. *PLoS ONE*, 2011, vol. 6, e18271
- **NISHIGUCHI A. et al.** Cell-cell crosslinking by biomolecular recognition of heparin-based layer-by-layer nanofilms. *Macromol Biosci.*, 2015, vol. 15, 312-317